# EUROPEAN PATENT APPLICATION

(11) **EP 2 523 247 A1**
(43) Date of publication of application: **14.11.2012**
(21) Application number: 12166942.8
(22) Date of filing: 07.05.2012
(51) Int. Cl.: H01M 10/0567, H01M 10/052, H01M 10/42

(54) **Additive for overcharge preventing of secondary battery and non-aqueous electrolyte for secondary battery including the same**

(30) Priority: 11.05.2011 KR 20110043999
(71) Applicant: Samsung Corning Precision Materials Co., Ltd., Gyeongsangbuk-do Gumi-si, 730-735 (KR)
(72) Inventor: Choi, Shin Jung, 336-841 Asan-si ChungCheongNam-Do (KR); Lee, Mi Sun, 336-841 Asan-si ChungCheongNam-Do (KR)
(74) Representative: Gulde Hengelhaupt Ziebig & Schneider

(57) **Abstract**

An additive for overcharge prevention of a lithium ion battery is provided. A non-aqueous electrolyte for a lithium ion battery including a lithium salt and an organic solvent is also provided, which includes both a terphenyl derivative and a xylene derivative. The additive undergoes oxidative polymerization during overcharge and increases resistance of an electrode surface, thereby shutting down overcharge current. When the additive of the present invention is used in the electrolyte of a lithium ion battery, the safety of battery can be improved during overcharge.

## Description

### Background of the Invention

### 1. Field of the Invention

Aspects of the present invention relate to an additive for overcharge prevention of a secondary battery, and more particularly, to an additive for overcharge prevention of a secondary battery, which includes both a terphenylene derivative and a xylene derivative, and a nonaqueous electrolyte for a secondary battery including the same.

### 2. Description of the Related Art

Recently, interests in energy storage technology have been gradually increased. As the use of batteries is enlarged to applications for the storage of energy for cell>rtlar phones, camcorders, notebook computers, personal computers and electric vehicles, efforts on the research and development of electrochemical devices are increasingly embodied and the field of electrochemical devices receives the greatest attention, and among them, interests in the development of chargeable/dischargeable secondary batteries are focused.

However, when the secondary battery is overcharged, the battery will undergo thermal runaway, leading to ignition and explosion due to successive exothermic decomposition reactions.

In attempts to control the above-described ignition or explosion resulting from temperature rise in the battery, many solutions have been suggested, and one example thereof is a method that uses nonaqueous electrolyte additives. However, when the additive for overcharge prevention (chloroanisole) is used, which undergoes oxidation-reduction cycling (redox shuttle) mechanism, has a problem in that it is not effective when the charge current of the battery is high. Also, when biphenyl or cyclohexylbenzene is used alone, the deterioration in the performance of the battery may undesirably occur

### Brief Summary of the Invention

Aspects of the present invention provide an additive for overcharge prevention of a secondary battery, which can improve the safety of a battery during overcharge of the battery without deteriorating the performance of the battery, and a nonaqueous electrolyte for a secondary battery including the additive.

In accordance with one aspect of the present invention, there is provided an additive for overcharge prevention of a secondary battery, the additive including a terphenyl derivative represented by the following formula (1), and a xylene derivative represented by the following formula (2): 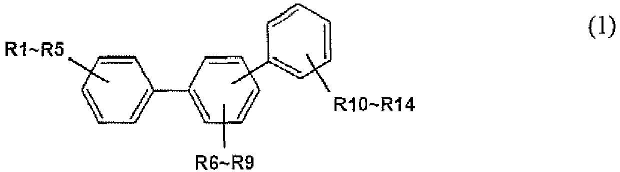 wherein R1 to R14 are independently selected from the group consisting of -H, -F, - Cl, -OH, -NO2, a C1 to C12 alkyl, a C1 to C12 aminoalkyl, a C1 to C12 hydroxyalkyl, a C1 to C12 haloalkyl, a C1 to C12 alkoxy, and a C3 to C8 cycloalkyl, and 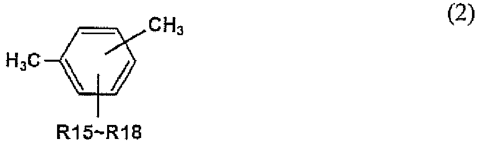 wherein R15 5 to R18 are independently selected from the group consisting of-H, -F, -Cl, -OH, -NO₂, a C1 to C12 alkyl, a C1 to C12 aminoalkyl, a C1 to C12 hydroxyalkyl, a C1 to C12 haloalkyl, a C1 to C12 alkoxy, and a C3 to C8 cycloalkyl.

In accordance with another aspect of the present invention, there is provided a nonaqueous electrolyte for overcharge prevention of a secondary battery, the nonaqueous electrolyte including the additive according to the present invention, a lithium salt, and an organic solvent.

As described above, when an additive for overcharge prevention of a secondary battery according to the present invention is used, overcharge current can be effectively prevented by forming a passivation film having increased resistance during overcharge without adding an excessive amount of the additive, thereby improving the safety of the battery without deteriorating the performance of the battery.

Additional aspects and/or advantages of the invention will be set forth in part in the description which follows and, in part, will be obvious from the description, or may be learned by practice of the invention.

### Brief Description of the Drawings

The objects, features and advantages of the present invention will be more apparent from the following detailed description in conjunction with the accompanying drawings, in which:
FIGS. 1A to 1E are views illustrating successive 10 times' cyclic voltammetry curves obtained from electrolytes including biphenyl, o-terphenyl, p-terphenyl, cyclohexylbenzene, and xylene, respectively;
FIGS. 2A to 2C are views illustrating successive 10 times' cyclic voltammetry curves obtained from electrolytes including 25 mM o-terphenyl + 75 mM xylene, 25 mM *o-*terphenyl + 75 mM cyclohexylbenzene, and 25 mM biphenyl + 75 mM cyclohexylbenzene, respectively;
FIG. 3A is a view illustrating impedance curves obtained at 5.0 V after forming a passivation film by applying successive 10 times' cyclic voltages to an electrolyte including biphenyl, o-terphenyl,p-terphenyl, cyclohexylbenzene, and xylene, and FIG. 3B is a view illustrating total resistance (Rₜₒₜ) of impedance curves obtained at 4.2 V, 4.6 V, 5.0 V, and 5.3 V;
FIGS. 4A to 4C are views illustrating impedance curves obtained at 5.0 V after forming passivation films by applying successive 10 times' cyclic voltages to electrolytes including 25 mM o-terphenyl + 75 mM xylene, 25 mM o-terpllenyl + 75 mM cyclohexylbenzene, and 25 mM biphenyl + 75 mM cyclohexylbenzene, respectively.

### Detailed Description of the Invention

Hereinafter, the present invention will be described.

The additive for overcharge prevention of a secondary battery according to the present invention includes a terphenyl derivative represented by the following formula (1); and
a xylene derivative represented by the following formula (2): 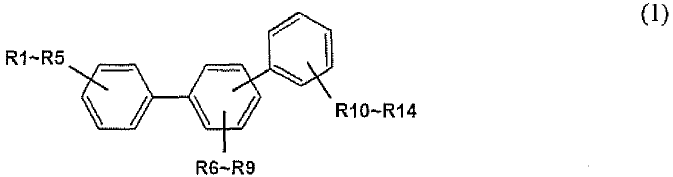 wherein R1 to R14 are independently selected from the group consisting of-H, -F, - C1, -OH, -NO2, a C1 to C12 alkyl, a C1 to C12 aminoalkyl, a C1 to C12 hydroxyalkyl, a C1 to C12 haloalkyl, a C1 to C12 alkoxy, and a C3 to C8 cycloalkyl; and 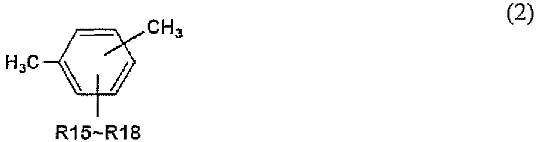 wherein R15 to R18 are independently selected from the group consisting of -H, -F, -Cl, -OH, -NO₂, a C1 to C12 alkyl, a C1 to C12 aminoalkyl, a C1 to C12 hydroxyalkyl, a C1 to C12 haloalkyl, a C1 to C12 alkoxy, and a C3 to C8 cycloalkyl.

According to an embodiment of the present invention, the terphenyl derivative is a compound oxidized at a voltage higher than the operating voltage of a positive electrode. The oxidation initiation voltage (vs. Li/Li⁺) of the terphenyl derivative is preferably higher than 4.4 V, and more preferably 4.5-4.7 V.

If the oxidation initiation voltage of the terphenyl derivative is lower than 4.4 V, the additive may form a film on the positive electrode by an electrochemical reaction during normal charging and discharging, thereby reducing the capacity of the battery. If the oxidation initiation voltage of the terphenyl derivative is higher than 4.7 V, a decomposition reaction of electrolyte may occur before passivation film formation, leading to explosion. Therefore, in order to rapidly prevent the flow of current during overcharge without affecting normal charging and discharging, it is preferable that the terphenyl derivative be in the above-described range.

Non-limiting examples of the terphenyl derivative include *o*-terphenyl, *m*-terphenyl, p-terphenyl, fluoroterphenyl, chloroterphenyl, cyclohexylterphenyl, methoxyterphenyl and so on. The content of the terphenyl derivative in the electrolyte is not specifically limited, but is preferably 0.1-10 parts by weight based on 100 parts by weight of the electrolyte. If the content of the terphenyl derivative is less than 0.1 parts by weight, it will have an insignificant effect on the prevention of overcharge, and if it is more than 10 parts by weight, it may cause deterioration in the performance of the battery.

According to an embodiment of the present invention, the xylene derivative is a compound oxidized at a voltage higher than the operating voltage of a positive electrode. The xylene derivative preferably has an oxidation initiation voltage in a range of 4.6 to 5.0 V (vs. Li/Li⁺), which is higher than the oxidation initiation voltage of the terphenyl derivative and lower than the oxidation initiation voltage of the electrolyte. This is for the purpose of forming a film having a large thickness and high resistance. That is to say, during overcharge, terphenyl is first oxidized to form a polyterphenyl having high conductivity, forming a thick film, and xylene derivative is then oxidized due to an increased voltage to form polyxylene having high resistance and small thickness, increasing the resistance of the film. Non-fimiting examples of the xylene, derivative include o-xylene, *m*-xylene, *p*-xylene, fluoroxylene, chloroxylene, ethylxylene, propylxylene, cyclohexylxylene, methoxyxylene, dimethoxyxylene, trimethoxyxylene, fluoromethoxyxylene, chlorometlioxyxylene and so on. In addition, compounds that can be oxidized in the above-described voltage range to cause action mechanisms simitar to the above mechanisms fall within the scope of the xylene according to the present invention. The content of the xylene derivative in the electrolyte is not specifically limited, but is preferably 0.1-10 parts by weight based on 100 parts by weight of the electrolyte. If the content of the xylene derivative is less than 0.1 parts by weight, it will have an insignificant effect on the prevention of overcharge, and if it is more than 10 parts by weight, it can cause deterioration in the performance of the battery.

The molar ratio of the terphenyl derivative to the xylene derivative may range from 9:1 to 1:9. Importantly, an overcharge prevention film should have high resistance. Accordingly, the molar ratio of the terphenyl derivative to the xylene derivative preferably ranges from 1:1 to 1:3.

If the molar ratio of the terphenyl derivative to the xylene derivative is beyond the above-described range, a thickness of the formed film is excessively small, so that the film may have the risk of deteriorating the safety of the battery.

The present invention also provides a nonaqueous electrolyte for overcharge prevention of a secondary battery, the nonaqueous electrolyte including a lithium salt, an organic solvent and an additive. Anions of the lithium salt include F⁻, Cl⁻, Br⁻, I⁻, NO₃⁻, N(CN)₂⁻, BF₄⁻, ClO₄⁻, PF₆⁻, (CF₃)₂PF₄⁻, (CF₃)₃PF₃⁻, (CF₃)₄PF₂⁻, (CF₃)₅PF⁻, (CF₃)₆P⁻, CF₃SO₃⁻, CF₃CF₂SO₃⁻, (FSO₂)₂N⁻, (CF₃SO₂)₂N⁻, (CF₃CF₂SO₂)₂N⁻, (CF₃SO₂)₂CH⁻, (SF₅)₃C⁻, (CF₃SO₂)₃C⁻, CF₃CO₂⁻, CH₃CO₂⁻ and SCN⁻. Non-limiting examples of the solvent that may be used include propylene carbonate, ethylenecarbonate, diethylcarbonate, dimethylcarbonate, dipropylcarbonate, ethylmethylcarbonate, ethylpropylcarbonate, methylpropylcarbonate, butylenecarbonate, dimethylsulfoxide, acetonitrile, dimethoxyethane, diethoxyethane, tetrahydrofuran, N-methyl-2-pyrrolidone, gamma-butyrolactone or a mixture thereof.

Since the nonaqueous electrolyte according to the present invention includes both a terphenyl derivative and a xylene derivative as additives, the safety of a secondary battery can be improved during overcharge without deteriorating the performance of the secondary battery. Even if the terphenyl derivative and the xylene derivative are added in small amounts, they may react with each other and may be oxidized earlier than a solvent of the electrolyte during overcharge, producing a polymerized product, which may serve as resistance that shuts off overcharge current. Therefore, no further overcharge occurs, thereby suppressing rapid heat generation, ignition or explosion.

Reference will now be made in detail to the preferred embodiments of the present invention. It is to be understood that the following examples are illustrative only and the present invention is not limited thereto.

### EXAMPLE 1

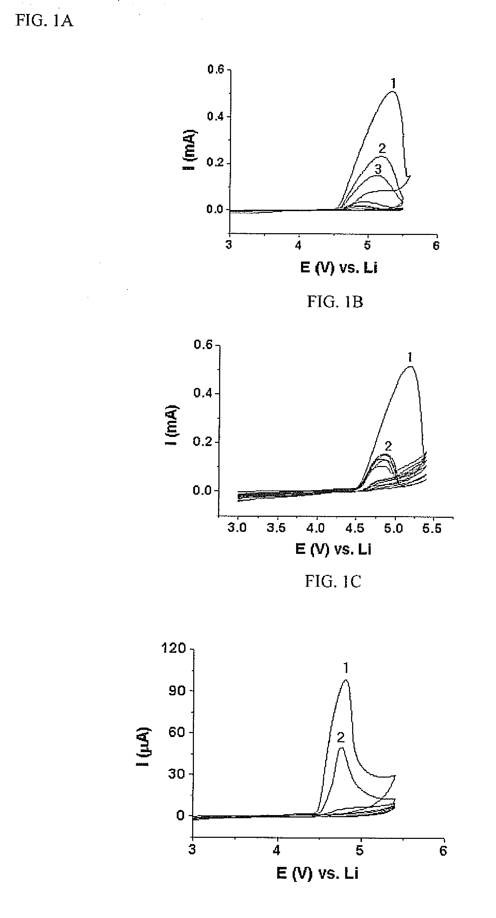

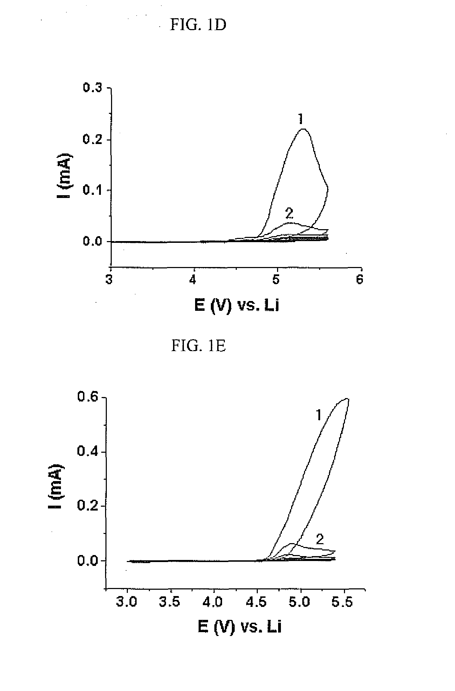

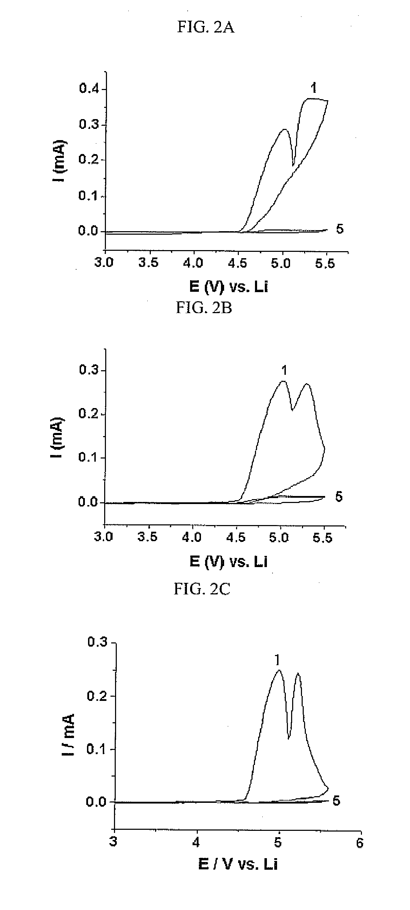

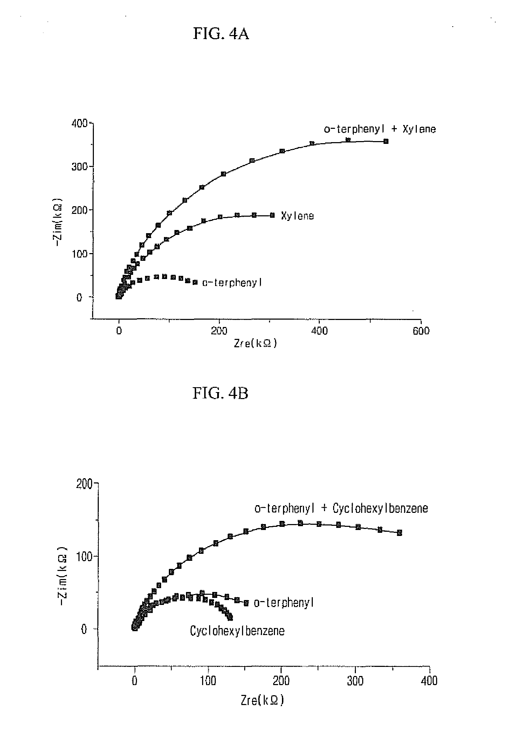

To a solution prepared by dissolving 1 M LiClO₄ in propylene carbonate were additives of 25 mM o-terphenyl and 75 mM xylene to prepare an electrolyte. Glassy carbon was used as a working electrode and a passivation film was formed by applying 10 times' cyclic voltages ranging from 3.0 V to 5.5 V at a rate of 50 mV/sec using EG&G Princeton Applied Research model 273A Potentiostat equipment operated by M270 software. Here, passivation film formation is confirmed from the cyclic voltammetry curves shown in FIG. 1. Then, impedance measurement was performed at 4.2 V, 4.6 V, 5.0 V, and 5.3 V using a Solartron SI 1255 HF frequency response analyzer operated by EG&G M398 EIS software. Here, the AC amplitude was ± 5 mV (peak-to-peak), and experimentation was carried out to collect 10 points per decade in a range of 100 kHz to 1 Hz. The impedance experiment result was analyzed using EG&G ZSimpWin software, impedance values were obtained from the analysis result, and the results are shown in FIGS. 2A and 4A.

### COMPARATIVE EXAMPLE 1

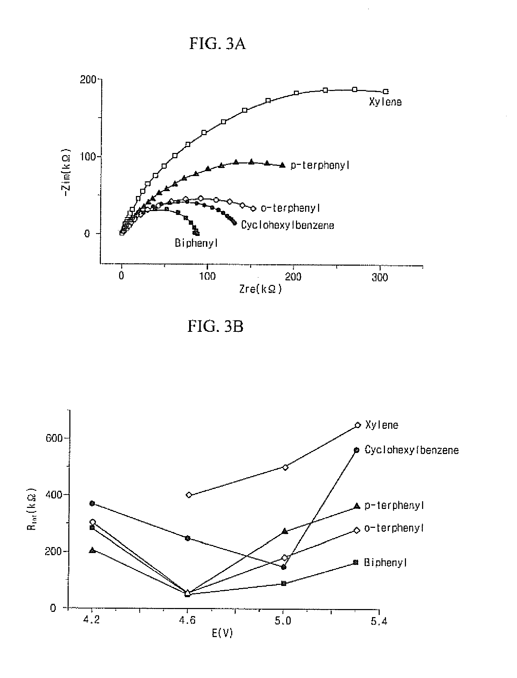

An electrolyte was prepared in the same manner as in Example 1, except that 100 mM biphenyl was used as an additive, and the results are shown in FIGS. 1A and 3.

### COMPARATIVE EXAMPLE 2

An electrolyte was prepared in the same manner as in Example 1, except that 100 mM *o*-terphenyl was used as an additive, and the results are shown in FIGS. 1B and 3.

### COMPARATIVE EXAMPLE 3

An electrolyte was prepared in the same manner as in Example 1, except that 30 mmp-terphenyl was used as an additive, and the results are shown in FIGS. 1C and 3.

### COMPARATIVE EXAMPLE 4

An electrolyte was prepared in the same manner as in Example 1, except that 100 mM cyclohexylbenzene was used as an additive, and the results are shown in FIGS. 1D and 3.

### COMPARATIVE EXAMPLE 5

An electrolyte was prepared in the same manner as in Example 1, except that 100 mM xylene was used as an additive, and the results are shown in FIGS. 1E and 3.

### COMPARATIVE EXAMPLE 6

An electrolyte was prepared in the same manner as in Example 1, except that 25 mM o-terphenyl and 75 mM cyclohexylbenzene were used as additives, and the results are shown in FIGS. 2B and 4B.

### COMPARATIVE EXAMPLE 7

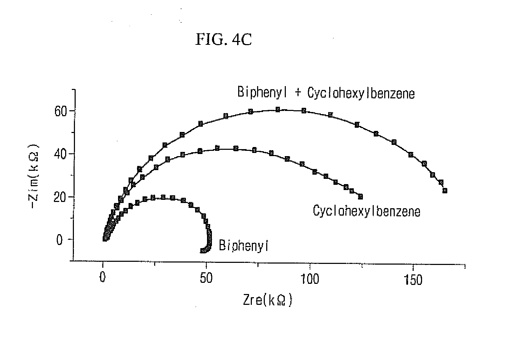

An electrolyte was prepared in the same manner as in Example 1, except that 25 mM biphenyl and 75 mM cyclohexylbenzene were used as additives, and the results are shown in FIGS. 2C and 4C.

FIGS. 1A to 1E illustrate cyclic voltammetry curves obtained when biphenyl derivative or benzene derivative were used alone. The biphenyl derivative starts to be oxidized at approximately 4.5 V, while the benzene derivative starts to be oxidized at approximately 4.7 V. As voltage cycles are repeated, a reduction in the peak current occurs in all cases shown in FIGS. 1A to 1E, suggesting that the resistance of a film formed by oxidative polymerization gradually increases. Therefore, the passivation film formation can be know from the graphical representation of the cyclic voltammetry curves. FIGS. 2A to 2C illustrate cyclic voltammetry curves obtained from nonaqueous electrolytes prepared in Example 1 and Comparative Examples 2 and 4 to 7, in which two current peaks are demonstrated, suggesting that oxidative polymerization of a terphenyl derivative or a biphenyl derivative occurs first and oxidative polymerization of a benzene derivative occurs then. Like in the cases shown in FIGS. 1A to 1E, as voltage cycles are repeated, a reduction in the peak current also occurs in all cases shown in FIGS. 2A to 2C and the resistance of a film formed by oxidative polymerization gradually increases,

Results of impedance experiments carried out on passivation films formed by cyclic voltammetry are shown in FIGS. 3 and 4. Referring to FIGS. 3 and 4, semi-circular or semi-oval curves are shown. Rₜₒₜ corresponds to the Zre-axis intercept obtained by extrapolation of the curves. Referring back to FIG. 3, the total resistance (Rₜₒₜ) of the passivation film made from each additive varies according to the voltage. The passivation film made from a benzene derivative generally demonstrates higher resistance than the passivation film made from terphenyl or biphenyl. Therefore, it is confirmed that the passivation film made from benzene derivative functions more effectively than the passivation film made from terphenyl or biphenyl.

FIGS. 4A to 4C illustrate impedance curves obtained from passivation films prepared in Example 1 and Comparative Examples 6 and 7. In all cases shown in FIGS. 4A to 4C, higher resistance is demonstrated than in a case where a terphenyl derivative, a biphenyl derivative or a benzene derivative is used alone.

During overcharge, even by using the same amount, the passivation films create a synergetic effect to function as effective resistance films. The reason of the foregoing is as follows. Since the oxidation initiation voltage of biphenyl or terphenyl is lower than that of benzene, the biphenyl or terphenyl derivative is first decomposed during overcharge, forming a thick film. However, since an oxidation film still has conductivity, the benzene derivative is oxidized in a subsequent step. While a film formed from electrochemical oxidative polymerization of the benzene derivative is relatively thin, the resistance of the film is considerably high, thereby effectively shutting down overcharge current. Referring to FIG. 4A, in a case where both *o*-terphenyl and xylene are used in combination, the resistance of the passivation film is higher than in a case where o-terphenyl or xylene is used alone. When both *o*-terphenyl and xylene are used, a thick film is formed using *o-*terphenyl and a high-resistance film is formed thereon using xylene, thereby forming a highly effective overcharge prevention film having a large thickness and high resistance. Here, a combination of terphenyl and cyclohexylbenzene or a combination of biphenyl and cyclohexylbenzene has lower resistance than a combination of terphenyl and xylene. Therefore, it is understood that when a combination of the terphenyl derivative and the xylene derivative according to the present invention is use in combination as an additive, the formed overcharge prevention film demonstrate relatively high performance.

Although exemplary embodiments of the present invention have been described in detail hereinabove, it should be understood that many variations and modifications of the basic inventive concept herein described, which may appear to those skilled in the art, will still fall within the spirit and scope of the exemplary embodiments of the present invention as defined by the appended claims.

## Claims

1. An additive for overcharge prevention of a secondary battery, the additive comprising:
a terphenyl derivative represented by the following formula (1); and
a xylene derivative represented by the following formula (2): 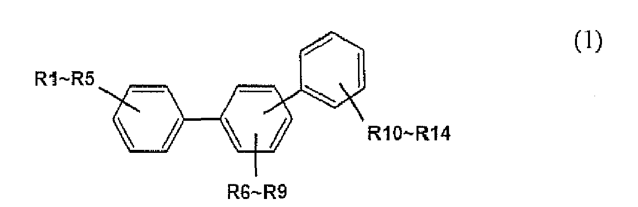 wherein R1 to R14 are independently selected from the group consisting of -H, -F, - Cl, -OH, -NO2, a C1 to C12 alkyl, a C1 to C12 aminoalkyl, a C1 to C12 hydroxyalkyl, a C1 to C12 haloalkyl, a C1 to C12 alkoxy, and a C3 to C8 cycloalkyl; and 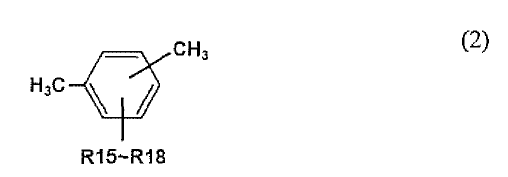 wherein R15 to R18 are independently selected from the group consisting of-H, -F, -Cl, -OH, -NO₂, a C1 to C12 alkyl, a C1 to C12 aminoalkyl, a C1 to C12 hydroxyalkyl, a C1 to C12 haloalkyl, a C1 to C12 alkoxy, and a C3 to C8 cycloalkyl.

2. The additive of claim 1, wherein the terphenyl derivative has an oxidation initiation voltage (vs. Li/L¡⁺) in a range of 4.4 to 4.7 V.

3. The additive of claim 1, wherein the xylene derivative has an oxidation initiation voltage in a range of 4.6 to 5.0 V.

4. The additive of claim 1, wherein the terphenyl derivative is at least one compound selected from the group consisting of *o*-terphenyl, *m*-terphenyl, *p*-terphenyl, fluoroterphenyl, chloroterphenyl, methylterphenyl, ethylterphenyl, propylterphenyl, butylterphenyl, cyclohexylterphenyl, methoxyterphenyl, dimethoxyterphenyl, fluoromethoxyterphenyl, chloromethoxyterphenyl, and methylmethoxyterphenyl.

5. The additive of claim 1, wherein the xylene derivative is at least one compound selected from the group consisting of o-xylene, *m*-xylene, *p*-xylene, fluoroxylene, chloroxylene, bromoxylene, ethylxylene, propylxylene, butylxylene, cyclohexylxylene, methoxyxylene, dimethoxyxylene, trimethoxyxylene, fluoromethoxyxylene, chloromethoxyxylene, and methylmethoxyxylene.

6. The additive of claim 1, wherein the terphenyl derivative is used in an amount of 0.1-10 parts by weight based on 100 parts by weight of the nonaqueous electrolyte.

7. The additive of claim 1, wherein the xylene derivative is used in an amount of 0.1-10 parts by weight based on 100 parts by weight of the nonaqueous electrolyte.

8. The additive of claim 1, wherein the molar ratio of the terphenyl derivative to the xylene derivative ranges from 1:1 to 1:3.

9. A nonaqueous electrolyte for overcharge prevention of a secondary battery, the nonaqueous electrolyte comprising:
the additive of any one of claims 1 to 8;
a lithium salt; and
an organic solvent.
